# EUROPÄISCHE PATENTANMELDUNG

(11) **EP 0 704 455 A1**
(43) Veröffentlichungstag der Anmeldung: **03.04.1996**
(21) Anmeldenummer: 95111039.4
(22) Anmeldetag: 14.07.1995
(51) Int. Cl.: C07H 15/04

(54) **Verfahren zur Bleichung von wässrigen Tensidlösungen**

(30) Priorität: 14.09.1994 DE 4432623
(71) Anmelder: HÜLS AKTIENGESELLSCHAFT, D-45764 Marl (DE)
(72) Erfinder: Grund, Gerda, Dr., D-49249 Dülmen (DE); Tanger, Uwe, Dr., D-44879 Bochum (DE); Grützke, Jürgen, D-44803 Bochum (DE); Schmidt, Stefan, Dr., D-45721 Haltern (DE)

(57) **Zusammenfassung**

Die Erfindung betrifft ein Verfahren zur Bleichung von wäßrigen Alkylpolyglycosidlösungen mit Wasserstoffperoxid, wobei in einem ersten Schritt die eigentliche Bleichung bei alkalischen pH-Werten in Gegenwart eines anorganischen Zusatzstoffes (Zersetzungsinhibitor für Wasserstoffperoxid) durchgeführt wird und in einem zweiten Schritt eine gezielte Zersetzung des nicht abreagierten Wasserstoffperoxids durch Kontakt mit Übergangsmetallen bzw. deren anorganischen Verbindungen erfolgt.

## Beschreibung

Die Erfindung betrifft ein Verfahren zur Bleichung von wäßrigen Tensidlösungen mit Wasserstoffperoxid, wobei in einem ersten Verfahrensschritt die eigentliche Bleichung bei alkalischen pH-Werten in Gegenwart anorganischer Zusatzstoffe, die als Zersetzungsinhibitoren für Wasserstoffperoxid wirken, durchgeführt wird und in einem zweiten Verfahrensschritt eine gezielte Zersetzung des nicht abreagierten Wasserstoffperoxids bzw. daraus gebildeter oxidierend wirkender Folgeprodukte durch Kontakt mit Übergangsmetallen bzw. deren anorganischen Verbindungen erfolgt.

Das Verfahren ist insbesondere interessant für Tensidlösungen, die herstellungsbedingt Verfärbungen enthalten, welche vor der Anwendung in Formulierungen für das Gebiet Waschen und Reinigen inklusiv der Personal-Care-Anwendungen zu hellfarbigen Produkten gebleicht werden müssen. Dies trifft insbesondere z. B. auf Alkylpolyglycoside (APG) zu.

Alkylpolyglycoside werden aus natürlichen Rohstoffen hergestellt und sind ungiftige und leicht abbaubare oberflächenaktive Stoffe. Sie werden deshalb als Wasch- und Reinigungsmittel und als Emulgatoren und Dispergatoren verwendet. Sie haben aber nur dann die gewünschten Grenzflächeneigenschaften, wenn die Alkylgruppen mindestens 8 C-Atome aufweisen. Die Alkylpolyglycoside im Sinne dieser Erfindung genügen der Formel

R' - O - Zₙ,

in der R' für einen linearen oder verzweigten, gesättigten oder ungesättigten aliphatischen Alkylrest mit 8 bis 18 Kohlenstoffatomen oder Gemische davon und Zₙ für einen Polyglycosylrest mit n = 1,1 bis 3 Hexose- oder Pentoseeinheiten oder Gemische davon stehen.

Bevorzugt werden Alkylpolyglycoside mit Alkylresten mit 12 bis 16 Kohlenstoffatomen sowie einem Polyglycosylrest mit n = 1,1 bis 2. Besonders bevorzugt werden Alkylpolyglycoside mit einem Polyglycosylrest mit n = 1,1 bis 1,4.
Als Polyglycosylrest wird der Polyglucosylrest bevorzugt.

Alkylpolyglycoside mit langkettigen Alkylgruppen werden im allgemeinen durch ein- oder mehrstufige Synthesen hergestellt. Ein einstufiges Herstellverfahren wird u. a. in DE-A-41 01 252 beschrieben.

Ein zweistufiges Herstellverfahren wird beispielsweise in EP-A-0 306 652 angegeben, wonach man zunächst durch Glycosidierung mit n-Butanol ein n-Butylglycosid und darauf durch Umglycosidierung mit einem langkettigen Alkohol das gewünschte langkettige Alkylpolyglycosid herstellt.

Nach beendeter Reaktion liegen die Alkylpolyglycoside in langkettigen Alkoholen gelöst vor. Anschließend müssen diese Alkohole abgetrennt werden, wenn man in Wasser klar lösliche Produkte erhalten will.

Die so erhaltenen wäßrigen Alkylpolyglycosid-Lösungen sind für hohe ästhetische Anforderungen noch zu dunkel und müssen in der Regel einer Bleichung unterzogen werden.

In der Literatur gibt es zahlreiche Hinweise, wie die Bleichung durchgeführt werden kann. So beschreibt Staley (EP 0 165 721) ein Verfahren zur Bleichung mit Wasserstoffperoxid, Schwefeldioxid, Ozon bzw. Persäuren. Dadurch, daß keinerlei Kontrolle bzw. Einstellung des pH-Werts vorgesehen ist, sind die Bleichergebnisse unbefriedigend. Außerdem können so in unerwünschtem Ausmaß Nebenprodukte entstehen, die z. T. auch einen ausgeprägten Eigengeruch haben.

Weitere Bleichmethoden, wie katalytische Hydrierung (US 4 762 918, Staley), Umsetzung mit Borhydrid (EP 0 388 857, Kao) und Bestrahlung mit UV-Licht (EP 0 526 710, Hüls) erwiesen sich als zu wenig wirksam.

Die Bleichung in Gegenwart von Bleichboostern, wie z. B. Erdalkaliionen bzw. Alkalisilicaten (WO 93 13 113, Henkel) unter Verwendung von Wasserstoffperoxid, liefert zwar deutlich bessere Bleichresultate, jedoch ist es schwierig, unter diesen Bedingungen wasserstoffperoxidfreie Produkte zu erhalten. Restmengen an Oxidationsmitteln sind auf jeden Fall zu vermeiden, da sie u. a. die Zersetzung von Zusatzstoffen in APG-haltigen Formulierungen bewirken. Der Vorschlag, das Problem der Rest-Wasserstoffperoxid-Bleichung in Gegenwart von Übergangsmetallverbindungen zu lösen (DE 42 18 073, Henkel), kann ebenfalls nicht überzeugen. Die dann beschleunigte katalytische Zersetzung des Wasserstoffperoxids zu Sauerstoff während der Bleichreaktion verringert die verfügbare Menge an Wasserstoffperoxid. So wird das Bleichergebnis spürbar verschlechtert und uneffektiver.

Es bestand daher die Aufgabe, die Bleichung so durchzuführen, daß in konzentrierten Tensidlösungen nach einem ersten oxidativen Schritt die restliche, nicht abreagierte Menge an Wasserstoffperoxid bzw. daraus entstandene oxidierend wirkende Folgeprodukte (z. B. Peroxide) auf einfache Weise unschädlich gemacht werden kann, ohne zwischendurch den ursprünglich zugesetzten Stabilisator zu entfernen.

Es wurde nun gefunden, daß eine effektive Bleichung, die zu wasserstoffperoxidfreien Tensidlösungen führt, möglich ist, wenn das Bleichverfahren in zwei Schritten durchgeführt wird, wobei in einem ersten Schritt die eigentliche Bleichung mit Wasserstoffperoxid erfolgt und in einem zweiten Schritt die Restperoxid-Zersetzung und die Zersetzung der oxidierend wirkenden Folgeprodukte mit Übergangsmetallen und/oder deren Verbindungen in Anwesenheit des ursprünglich zugesetzten Stabilisators erfolgt.

Gegenstand der Erfindung ist daher ein Verfahren zur Bleichung von wäßrigen, konzentrierten Tensidlösungen, welches dadurch gekennzeichnet ist, daß in einem ersten Schritt die Bleichung der Tensidlösungen in Gegenwart eines anorganischen Zersetzungsinhibitors für Wasserstoffperoxid durchgeführt wird und in einem zweiten Schritt eine gezielte Zersetzung des nicht abreagierten Wasserstoffperoxids und/oder daraus entstandener oxidierend wirkender Folgeprodukte in Anwesenheit des Zersetzungsinhibitors durch Übergangsmetalle und/oder deren Verbindungen erfolgt.

Völlig überraschend ist dabei, daß die zunächst für die Durchführung des ersten Bleichschritts gewünschte Stabilisatorwirkung der Zersetzungsinhibitoren im zweiten Schritt zu keiner Behinderung der Zersetzung führt und daher die Zersetzung der unerwünschten Rest- und Folgeprodukte keinesfalls stört.

Der erste Schritt der Bleichung, z. B. einer Alkylpolyglycosidlösung, erfolgt vorzugsweise kontinuierlich im Rührreaktor, im Rohrreaktor oder in einer Kombination aus Rührreaktor und Rohrreaktor, wobei die Reihenfolge beliebig ist. Es kann aber auch eine Rührkesselkaskade verwendet werden. Möglich ist auch eine diskontinuierliche Bleichung im Rührreaktor (Batch-Fahrweise).

Bei der Bleichung beträgt die Konzentration der wäßrigen Alkylpolyglycosid-Lösung 30 bis 75 Gewichtsprozent. Die verwendete Wasserstoffperoxidmenge beträgt 0,1 bis 7, vorzugsweise 0,5 bis 4 Gewichtsprozent (bezogen auf Trockensubstanz). Die Temperatur bei der Bleichung beträgt 40 bis 95 °C, wobei Temperaturen von 50 bis 80 °C besonders bevorzugt werden. Der pH-Wert beträgt 7 bis 12, vorzugsweise 8 bis 11, und die Konzentration an Stabilisierungsmittel (Zersetzungsinhibitor) 50 bis 10 000, vorzugsweise 200 bis 6 000 ppm (bezogen auf die Trockensubstanz). Als Stabilisierungsmittel für das Wasserstoffperoxid werden vorzugsweise anorganische Magnesiumverbindungen eingesetzt.

Der zweite Schritt der Bleichung, die Zersetzung restlicher Mengen an Wasserstoffperoxid und/oder oxidierend wirkender Folgeprodukte, erfolgt mit Übergangsmetallen und/oder deren Verbindungen vorzugsweise kontinuierlich mit Durchleitung des Stoffstroms durch einen Rohrreaktor gefüllt mit festem Katalysator, gegebenenfalls auf einem Träger. Der Katalysator kann auch in Form einer Metallsalz-Lösung zudosiert werden, wobei in diesem Fall statt eines Rohrreaktors ein Rührreaktor verwendet werden kann.

Als Übergangsmetalle können Chrom, Mangan, Eisen, Cobalt, Nickel, Kupfer, Palladium oder Platin und deren Mischungen oder Legierungen verwendet werden. Die Übergangsmetallverbindungen können Salze des Chroms, Mangans, Eisens, Cobalts, Nickels, Kupfers, Palladiums oder Platins und deren Mischungen sein.

Als Salze kommen vor allem Halogenide, Sulfate, Oxide sowie Hydroxide der erwähnten Metalle in Betracht.

Als Trägermaterialien für die Übergangsmetalle bzw. für wasserunlösliche Übergangsmetallverbindungen sind vor allem Aktivkohle, Aluminiumoxid und Siliciumdioxid geeignet.

Die Partikelgröße im Festbett kann zwischen 0,5 mm und 5 cm liegen.

Die Metalle können neben dem Einsatz in pulverisierter Form auf Trägermaterial auch z. B. als Drahtnetze, Drehspäne, Metallwolle oder Blechkonstruktionen mit großer Oberfläche verwendet werden.

Die Temperatur bei der Zersetzung im zweiten Schritt der Bleichung beträgt 20 bis 150 °C, vorzugsweise 30 bis 120 °C, besonders bevorzugt wird der Temperaturbereich von 40 bis 100 °C.
Der pH-Wert wird gegenüber dem ersten Schritt der Bleichung im Normalfall nicht geändert. Er beträgt ebenfalls 7 bis 12, vorzugsweise 8 bis 11. Er kann aber auch gegebenenfalls innerhalb dieses Bereichs neu eingestellt werden und daher von dem pH-Wert des ersten Schritts abweichen.

Das Verfahren besitzt folgende Vorteile:
- die Peroxid-Zersetzung erfolgt unter milden Bedingungen (keine Gefahr der Produktschädigung),
- die Zersetzungsreaktion ist gut beherrschbar (kein starkes Schäumen durch anfangs zu intensive Sauerstoff-Entwicklung),
- es genügen bei entsprechenden Reaktionsbedingungen kurze Verweilzeiten für die vollständige Peroxid-Zersetzung,
- es entstehen sehr geruchsarme Produkte mit extrem heller Farbe,
- die Produkte sind sicher Wasserstoffperoxid- und Peroxid-frei,
- die Produkte haben bei Erwärmen eine hohe Farbstabilität,
- das Verfahren ist allgemein anwendbar auf herstellungsbedingt dunkel gefärbte Tensidlösungen, wie z. B. von anionischen Tensiden (Beispiele: Paraffinsulfonate, α-Sulfofettsäuremethylester) und nichtionischen Tensiden (Beispiel: APG).

Der ersten und/oder der zweiten Stufe des Bleichprozesses kann eine kontinuierlich arbeitende Entschäumermaschine nachgeschaltet sein, in der das u. U. schaumdurchsetzte APG durch Zentrifugieren verdichtet und so besser pumpbar gemacht wird.

## Patentansprüche

1. Verfahren zur Bleichung von wäßrigen, konzentrierten Tendsidlösungen,
dadurch gekennzeichnet,
daß in einem ersten Schritt die Bleichung der Tensidlösungen bei alkalischen pH-Werten mit Wasserstoffperoxid in Gegenwart eines anorganischen Zersetzungsinhibitors für Wasserstoffperoxid durchgeführt wird und in einem zweiten Schritt eine gezielte Zersetzung des nicht abreagierten Wasserstoffperoxids und/oder daraus entstandener oxidierend wirkender Folgeprodukte in Anwesenheit des Zersetzungsinhibitors durch Übergangsmetalle und/oder deren Verbindungen erfolgt.

2. Verfahren zur Bleichung von wäßrigen, konzentrierten Tensidlösungen nach Anspruch 1,
dadurch gekennzeichnet,
daß die Tenside nichtionische Tenside sind.

3. Verfahren zur Bleichung von wäßrigen, konzentrierten Tensidlösungen nach Anspruch 1,
dadurch gekennzeichnet,
daß die Tenside Alkylpolyglycoside sind.

4. Verfahren zur Bleichung von wäßrigen, konzentrierten Tensidlösungen nach Anspruch 1,
dadurch gekennzeichnet,
daß als Zersetzungsinhibitoren anorganische Magnesiumverbindungen eingesetzt werden.

5. Verfahren zur Bleichung von wäßrigen, konzentrierten Tensidlösungen nach Anspruch 1,
dadurch gekennzeichnet,
daß als Übergangsmetalle Chrom, Mangan, Eisen, Cobalt, Nickel, Kupfer, Palladium oder Platin oder deren Mischungen oder Legierungen eingesetzt werden.

6. Verfahren zur Bleichung von wäßrigen, konzentrierten Tensidlösungen nach Anspruch 1,
dadurch gekennzeichnet,
daß als Übergangsmetallverbindungen anorganische Salze des Chroms, Mangans, Eisens, Cobalts, Nickels, Kupfers, Palladiums oder Platins oder deren Mischungen eingesetzt werden.

7. Verfahren zur Bleichung von wäßrigen, konzentrierten Tensidlösungen nach Anspruch 1,
dadurch gekennzeichnet,
daß die Übergangsmetalle oder Übergangsmetallverbindungen auf Aluminiumoxid, Aktivkohle oder Siliciumdioxid als Trägermaterial aufgebracht sind.

8. Verfahren zur Bleichung von wäßrigen, konzentrierten Tensidlösungen nach Anspruch 1,
dadurch gekennzeichnet,
daß die Temperatur bei der Zersetzung der restlichen oxidierend wirkenden Verbindungen 20 bis 150 °C beträgt.

9. Verfahren zur Bleichung von wäßrigen, konzentrierten Tensidlösungen nach Anspruch 1,
dadurch gekennzeichnet,
daß der pH-Wert 7 bis 12 beträgt.

10. Verfahren zur Bleichung von wäßrigen, konzentrierten Tensidlösungen nach Anspruch 1,
dadurch gekennzeichnet,
daß die Zersetzung in einem Rohrreaktor erfolgt.
